Europäisches Patentamt

European Patent Office

Office européen des brevets

(19)

(11) Veröffentlichungsnummer : **0 108 215**
**B1**

(12) **EUROPÄISCHE PATENTSCHRIFT**

(45) Veröffentlichungstag der Patentschrift :
30.09.87

(51) Int. Cl.⁴ : **A 61 F   7/00**

(21) Anmeldenummer : 83108823.2

(22) Anmeldetag : 07.09.83

(54) Wärmepackung und Verfahren zur Herstellung einer Wärmepackung.

(30) Priorität : 09.09.82 CH 5356/82

(43) Veröffentlichungstag der Anmeldung :
16.05.84 Patentblatt 84/20

(45) Bekanntmachung des Hinweises auf die Patenterteilung : 30.09.87 Patentblatt 87/40

(84) Benannte Vertragsstaaten :
AT BE CH DE FR GB IT LI LU NL SE

(56) Entgegenhaltungen :
EP-A- 0 071 211
CH-A-    446 612
CH-A-    461 023
DE-A- 1 566 322
DE-A- 1 929 722
DE-A- 2 301 821
DE-A- 2 808 850
GB-A-      16 266
US-A- 3 122 142

(73) Patentinhaber : Tesch, Günter Horst
Avenue Jean-Marie-Musy 15
CH-1700 Fribourg (CH)

(72) Erfinder : Tesch, Günter Horst
Avenue Jean-Marie-Musy 15
CH-1700 Fribourg (CH)

(74) Vertreter : Lesser, Karl-Boiko, Dipl.-Ing.
European Patent Attorney Johanneskirchnerstrasse
149a
D-8000 München 81 (DE)

## Beschreibung

Die Erfindung betrifft eine Wärmepackung gemäß Oberbegriff des Anspruches 1 und ein Verfahren zur Herstellung einer Wärmepackung gemäß Oberbegriff des Anspruches 25.

Eine gattungsgemäße Wärmepackung und ein Verfahren zur Herstellung einer solchen Wärmepackung ist aus der DE-OS 2.301.821 bekannt. In dieser bekannten Wärmepackung ist eine erdartige Masse zwischen zwei die Masse umhüllenden Schichten gelagert. Die erdartige Masse besteht dort aus einem wässrigen Moorbrei. Die eine diese Masse umgebende Schicht, die mindestens für Flüssigkeit durchlässig ist, wird dort von einem netzartigen, großmaschigem Gewebe gebildet, das aufgrund seiner Durchlässigkeit einen direkten Kontakt der Moorbreilage mit dem zu behandelnden Körperteil gewährleistet. Zur Anwendung wird diese bekannte Wärmepackung nämlich direkt mit dem netzartigen Gewebe auf den zu behandelnden Körperteil aufgelegt. Die zweite, die Masse umgebende Schicht wird dort von einer Metallfolie gebildet, die von einer Wasser undurchlässigen Plastikfolie auf ihrer von der Moorbreilage abgewandten Seite getragen wird.

Bei dieser bekannten Wärmepackung sind nun eine Reihe weiterer Schichten vorgesehen. So liegt auf der zuletzt genannten Plastikfolie eine Wärmedämmschicht auf, die z. B. aus einer Moltopren-Schaumstoffschicht bestehen kann und die mit einer weiteren Plastikfolie bedeckt ist. Beide Wasser undurchlässigen Plastikfolien sind im Bereich ihrer Ränder miteinander verschweißt, sodaß die Schaumstoffschicht wasserdicht eingeschlossen ist. Auf der gegenüberliegenden Seite dieses Schichtensystems liegt nun auf dem netzartigen Gewebe eine aufklapp- bzw. entfernbare Abdeckfolie auf, die ihrerseits von einer Schutzfolie bedeckt wird. Alle Folien und das netzartige Gewebe sind am die Wärmepackung umlaufenden Rand miteinander verbunden, wobei die Verbindung der Schutzfolie mit den anderen Folien, insbesondere der außenliegenden obengenannten Plastikfolie an drei Kanten der Packung lösbar und wieder verbindbar ist. Diese bekannte Wärmepackung läßt sich in heißem Wasser erwärmen. Bei der ersten Anwendung der Packung wird die Schutzfolie an den vorgenannten drei Kanten gelöst und weggeklappt, worauf die das Gewebe abdeckende Abdeckfolie an Aufreißlinien aufgetrennt und gegebenenfalls vollkommen von der Packung abgetrennt wird. Die Packung wird dann mit dem netzartigen Gewebe auf den zu behandelnden Körperteil aufgelegt, wobei der Moorbrei durch den Auflagedruck durch die Maschen des Gewebes etwas hindurchdringt und damit auf der Haut des zu behandelnden Körperteils direkt aufliegt. Nach der Anwendung wird diese Wärmepackung mittels der Schutzfolie wieder geschlossen, wofür ein an den drei vorgenannten Kanten angeordneter Druckverschluß vorgesehen ist.

Der Aufbau und die Anwendung dieser bekannten Wärmepackung sind relativ kompliziert, insbesondere ist die Anwendung dieser bekannten Wärmepackung auf die Warmbehandlung mit wäßrigem Moorbrei beschränkt. Ein weiterer Nachteil dieser bekannten Wärmepackung besteht darin, daß dann, wenn auf diese Packung ein teilflächiger Druck aufgebracht wird, der Moorbrei an dieser Stelle verdrängt wird und sich an den benachbarten Bereichen anreichert. Es ist somit sehr schwierig, die Schichtdicke des Moorbreis zu kontrollieren.

Bei einer Wärmepackung, in der im wesentlichen nur Moorbrei vorgesehen ist, ändert sich über die Anwendungszeit der Packung die Wärmeabgabe, da der Moorbrei allmählich abkühlt.

Es sind nun Schmelzmassen bekannt (vgl. z. B. DE-A-2.808.850), die für Wärmebehandlungen benutzt werden können und die z. T. auch mit erdartiger Masse, wie Moor oder Fango, gemischt sind. Die bekannten Moor-Paraffin- bzw. Fango-Paraffin-Packungen können nur professionell von Masseuren und/oder Ärzten benutzt werden. Diese bekannten Packungen werden in einem Wärmeofen erhitzt, bis sie dünnflüssig sind. Diese Flüssigkeit wird auf eine Folie, z. B. aus Plastik, gebracht und erstarrt dort allmählich von der Außenseite der Schmelzmasse her, während die Packung innen immer noch flüssig bleibt. Die äußerlich erstarrte Packung wird sofort vorsichtig auf den Patienten gelegt. Während der Anwendung auf dem Patienten erstarrt allmählich auch das Innere der Packung. Dabei bleibt während der gesamten Erstarrungszeit die Packung auf einem bestimmten Temperaturbereich und gibt die Erstarrungswärme ab. Solange diese bekannte Packung noch nicht völlig erstarrt ist, darf auf sie kein Druck ausgeübt werden, da sonst die äußerliche, schon erstarrte Schicht der ansonsten homogenen Packung platzt und die Flüssigkeit austritt.

Bei diesen bekannten Paraffinpackungen ist es auch nicht möglich, exakt eine bestimmte Schichtdicke über die gesamte Fläche der Packung einzuhalten.

Weiterhin sind Paraffinpackungen bekannt, bei denen das Paraffin in eine gas- und flüssigkeitsdichte Plastikfolie eingeschweißt ist, mit denen allein die Wärmeabgabe dieser Packung ausgenutzt werden kann. Wird bei dieser Packung ein Druck auf dieselbe ausgeübt, so ändert sich auch hierbei die Schichtdicke derselben, weshalb die Wärmeabgabe ungleichmäßig erfolgt.

Aus der älteren EP-A-0.071.211 ist ein der Körperbehandlung dienender Schichtkörper bekannt, der als Stand der Technik gemäß EPÜ Artikel 54 Absatz 3 und Artikel 56 gilt. Dieser Schichtkörper kann als Wärmepackung benutzt werden. Er weist zwischen zwei Außenschichten eine Gesteinspartikel, wie Sand, Splitt od. dgl., aber auch Lehm als sandhaltigen Ton oder Talcum, enthaltende Zwischenschicht auf, wobei alle drei Schichten miteinander vernadelt sind. Eine die abgegebene Wärme über einen längeren Zeitraum konstant haltende Schmelzmasse od. dgl. ist dieser Wärmepackung jedoch nicht beigegeben.

Der Erfindung liegt deshalb die Aufgabe zugrunde, eine gattungsgemäße Wärmepackung zu schaffen, die einfach anzuwenden ist, die über die gesamte Anwendungszeit eine gleich große Wärmeabgabe gewährleistet und deren Dicke sich auch bei Anwendung von teilflächigem Druck nicht ändert.

Diese Aufgabe wird durch den Gegenstand des Anspruches 1 gelöst. Erfindungsgemäß liegt bei einer gattungsgemäßen Wärmepackung zwischen den beiden Außenschichten eine Schicht aus erdartiger Masse und Schmelzmasse aus synthetischen und/oder natürlichen Kohlenwasserstoffwachsen vor und weiterhin sind diese drei flächigen Schichten durch vollflächig, verteilte Haltefasern durch die innere Schicht hindurch miteinander verbunden. Es wird dadurch eine Wärmepackung zur Verfügung gestellt, bei der die Haltefasern ein Verschieben der erdartigen Masse und auch der Schmelzmasse in der Fläche der Schicht verhindern und zwar einerseits dadurch, daß sie den beiden Massen bei einer solchen Bewegung im Wege stehen und andererseits die Schichtdicke dadurch, daß die beiden Außenschichten über die Haltefasern miteinander verbunden sind, nicht vergrößert werden kann. Gemäß einer bevorzugten Ausführungsform sind die drei Schichten miteinander vernadelt, wobei das Vernadeln dieser drei Schichten auf einer herkömmlichen Nadelvliesmaschine vorgenommen werden kann.

Als erdartige Masse kann Moor, Fango, Lehm, insbesondere in der Form von Heilerde, oder dergleichen verwendet werden. Unter erdartiger Masse sollen aber auch Füllstoffe wie Talkum, Kreide, Steatit und Sandpulver verstanden werden. Diese zuletzt genannten Stoffe haben zwar keine wesentliche Heilwirkung, sie dienen aber als weiterer Wärmespeicher und Verdicker für die Schmelzmasse. Diese können in getrockneter Form vorliegen.

Als Schmelzmasse eignen sich reine Paraffine oder Gemische von Paraffinen, deren Schmelzpunkte sich nur unwesentlich voneinander unterscheiden.

Die zwischen den beiden Außenschichten liegende innere Schicht besteht aus 30-70 Gewichtsprozent erdartiger Masse und aus 70-30 Gewichtsprozent Schmelzmasse, wobei vorzugsweise ein Mischungsverhältnis von 1 : 1 vorliegt. Dient die erdartige Masse nur als weiterer Wärmeträger und Verdicker, so kann ihr Anteil an der inneren Schicht viel kleiner sein, als der Paraffinanteil z. B. auch unter 10 % liegen. Insbesondere der erdartigen Masse können noch Wirkstoffe in flüssiger Form oder in Form von Pulvern, wie Öle, Schwefel, Pflanzenextrakte oder Heilkräuter beigegeben werden, während die Schmelzmasse noch Mittel zur Verhinderung des Ausblutens und zur Anpassung der Viskosität, insbesondere Verdicker aufweisen kann.

Die aus der erdartigen Masse und der Schmelzmasse bestehende innere Schicht liegt zwischen den beiden Außenschichten mit einem Flächengewicht von 3-30 kg pro m², vorzugsweise mit 8-15 kg pro m² vor.

Insbesondere dann, wenn die drei Schichten miteinander vernadelt sind, besteht mindestens die eine Außenschicht aus einem aktiv nadelfähigen Faservlies, welches z. B. ein Flächengewicht von 20-350 g/m², vorzugsweise von 100-180 g/m² aufweist. Dieses Faservlies ist gemäß einer bevorzugten Ausführungsform vorvernadelt und mit einem großmaschigen Fadengelege oder -gewebe verstärkt.

Gemäß einer Ausführungsform kann auch die andere Außenschicht aus einem Faservlies bestehen und z. B. ein Flächengewicht von 100-200 g pro m² aufweisen, die zweite Außenschicht kann aber auch aus einer Folie bestehen, die vorzugsweise eine Flächengewicht von 50-200 g pro m² aufweist. Diese Folie kann eine Aluminiumfolie sein, insbesondere aber eine zweischichtige Folie, deren eine Schicht aus Kunststoff und deren andere Schicht aus Aluminium besteht. Anstelle der Folie kann die zweite Außenschicht auch aus einem Vliesstoff, einer Gewebebahn oder einem Faserverbundstoff bestehen.

Gemäß einer besonderen Ausführungsform ist diese Folie eine sogenannte Näpfchenfolie, d. h. sie weist näpfchen- oder topfförmige Vertiefungen auf.

In diesen näpfchenförmigen Vertiefungen kann die Konzentration der Schmelzmasse gegenüber der erdartigen Masse größer sein, als in der übrigen inneren Schicht.

Die erdartige Masse und die Schmelzmase können nun in verschiedener Form zwischen den beiden Außenschichten vorliegen.

Gemäß einer Ausführungsform der Erfindung liegt ein Gemisch aus erdartiger Masse und Schmelzmasse vor, welches dadurch hergestellt wurde, daß die getrocknete und zerkleinerte erdartige Masse in eine Schmelze aus der Schmelzmasse eingerührt wird, wobei ein inniges Vermischen der beiden Massen erfolgt. Bei der fertigen Wärmepackung liegt dabei eine innere Schicht vor, die im wesentlichen vollflächig durchgehend ist und nur von den Haltefasern durchdrungen wird. Es ergibt sich somit eine sehr homogene innere Schicht. Die erdartige Masse ist somit in die Schmelzmasse eingebettet.

Gemäß einer anderen Ausführungsform liegt sowohl die erdartige Masse, als auch die Schmelzmasse in körniger Form vor. Die getrocknete erdartige Masse wurde z. B. vorher gemahlen, während die Schmelzmasse aus granulierten, gegossenen, geraspelten oder dergleichen Partikeln bestehen kann, die vorzugsweise unregelmäßige Gestalt aufweisen und deren kleinste Breite unter 1 mm und deren größte Länge im Bereich von 5-10 mm liegen kann.

In der inneren Schicht kann aber auch die Schmelzmasse als homogene Schicht vorliegen, während die erdartige Masse in trockener und körniger Form zwischen einer wasserdurchlässigen Außenschicht und der Schmelzmasse angeordnet ist. Diese Ausführungsform hat den Vorteil, daß aus der körnigen, erdartigen Masse bei der Anwendung der Wärmepackung Wirkstoffe durch die Außenschicht auf den Körper des Patienten herausdringen können. Die extra vorgesehene Schicht mit der Schmelzmasse liefert dann nur Wärme, die erst die erdartige Masse durchdringen muß, bevor sie auf den Körper des Patienten einwirkt.

Aus Vorgenanntem ergibt sich, daß die innere Schicht aus erdartiger Masse und Schmelzmasse verschieden aufgebaut sein kann, je nachdem welche Zwecke man mit dieser inneren Schicht verfolgt und welche Art der Heilung beim Patienten angewandt werden soll. So ist es auch vorstellbar, bei einer Wärmepackung, in der sowohl die erdartige Masse, als auch die Schmelzmasse in körniger Form vorliegt, eine Schichtentrennung zwischen diesen beiden vorzunehmen, so daß auf einer Seite der Wärmepackung der Anteil der erdartigen Masse größer ist, als auf der anderen Seite der inneren Schicht. Eine solche ungleiche Verteilung empfiehlt sich insbesondere dann, wenn eine der beiden Außenschichten aus einer Kunststoffolie besteht, wobei in deren Nähe dann die Schmelzmasse in größerer oder alleiniger Konzentration vorliegen sollte. Dies trägt dem Umstand Rechnung, daß die Schmelzmasse im wesentlichen nur als Wärmespeicher und -spenderdient, während die erdartigen Massen neben der Wärmelieferung auch Wirkstoffe an die zu beheilenden Körperteile abgeben sollen.

Es sei noch darauf verwiesen, daß insbesondere durch das Vernadeln des Schichtkörpers und der Bildung der Haltefasern die beiden Außenschichten aufeinanderzu gezogen werden, so daß diese einen Druck auf die innere Schicht ausüben.

Um die Anwendung der aus den beiden Außenschichten und der inneren Schicht gebildeten Wärmepackung zu erleichtern, ist diese zwischen zwei gas- und flüssigkeitsdichten Folien angeordnet, wobei diese Folien an ihren Rändern abgedichtet miteinander verbunden sind. Diese beiden, an ihren Rändern miteinander verbundenen Folien bilden nicht nur eine Transportverpackung, sondern sie gestatten auch, eine trockene, d. h. wasserfreie Anwendung der Packung. Die verpackte Wärmepackung kann in einem Wasserbad erwärmt werden, ohne daß die Wärmepackung selbst feucht wird. Zu diesem Zwecke wird die Wärmepackung mitsamt der Verpackung längere Zeit in kochendes Wasser gelegt, bis die gesamte Schmelzmasse geschmolzen ist. Je nach Dicke der Schmelzmasse in der Wärmepackung ist diese Zeit unterschiedlich, wobei in der Regel 15 Minuten als ausreichend angesehen werden können. Sind die beiden Folien zumindestens über einen Teil ihres Randes mit einem Druckverschluß versehen, so kann die Heilpackung auch nach Öffnen des Verschlußes und Anwendung derselben wieder verpackt werden.

Insbesondere für den Transport und längere Lagerzeit einer neuen, unbenutzten Heilpackung ist es vorteilhaft, daß innerhalb der die Wärmepackung aufnehmenden Verpackung ein Vakuum vorliegt. Auch wenn die benutzte Wärmepackung wieder zwischen die einen Beutel bildenden Folien gebracht wird, sollte die Luft aus diesem Beutel möglichst vollständig entfernt werden, wozu man bei der erfindungsgemäßen Wärmepackung auch einen punktuellen oder linienförmigen Druck auf diese ausüben kann, ohne daß sich die in der Wärmepackung vorliegende Masse zwischen den beiden sie umgebenden Schichten verschiebt. Ein solches Drücken ist bei der obenbeschriebenen bekannten Wärmepackung nicht möglich, weshalb in einer solchen bekannten, benutzten Packung auch dann, wenn sie mit der Schutzfolie geschlossen wird, sehr viel Luft verbleibt, durch die die erdartige Masse allmählich verdorben wird.

Gemäß einer besonderen Ausführungsform weisen die umlaufenden Ränder der aus den beiden Außenschichten und der Gemischschicht gebildeten Wärmepackung eine geringere Dicke auf, als der Rest der Wärmepackung, wobei im Bereich dieser Ränder die Schmelzmasse besonders stark in die Außenschichten eindringt. Man erhält dadurch nicht nur eine ansprechendere Form der Wärmepackung, die Ränder der Packung werden dadurch auch quasi-versiegelt.

Bei einer Wärmepackung, bei der zumindest die erdartige Masse in körniger Form getrennt von der Schmelzmasse vorliegt, kann die Wärmepackung, nachdem sie aus der Verpackung entnommen ist, auch in heißem Wasserdampf hängend erwärmt werden, so daß die Packung selbst nicht nur heiß wird, sondern darüberhinaus auch noch Wasser aufnimmt. Durch die Zwischenräume zwischen den Partikeln dringt das Wasser bzw. der Dampf sehr gut ein, darüberhinaus wird das Eindringen noch durch die Haltefasern erleichtert, die einen Kapillareffekt bewirken. Ebenso wie Wasser können vor der Anwendung noch die obengenannten Wirkstoffe eingebracht werden.

Die beiden Außenschichten verhindern insbesondere dann, wenn der Schmelzmasse noch Verdicker beigegeben ist und diese Masse mit der erdartigen Masse vermischt vorliegt, ein Austreten der Schmelzmasse auch dann, wenn diese verflüssigt ist.

Die erhitzte Wärmepackung kann auf den Körper aufgelegt werden, wobei die Kristallisationswärme der Schmelzmasse für die Wärmebehandlung des zu behandelnden Körperteils ausgenutzt wird. Dieser Effekt und die diesbezügliche Anwendung ist als solches, wie eingangs beschrieben, bekannt. Durch die erfindungsgemäße Ausbildung der Wärmepackung kann diese gegenüber den bekannten Moor-, Paraffin- bzw. Fango-Paraffin-Packungen nun von jedermann auf einfachste Art und Weise benutzt werden, ohne daß es spezieller Geräte bedarf.

Ein Verfahren zur Herstellung einer Wärmepackung gemäß Oberbegriff des Anspruches 25 besteht darin, eine Schicht aus erdartiger Masse und Schmelzmasse aus synthetischen und/oder natürlichen Kohlenwasserstoffwachsen zwischen den beiden Außenschichten einzubringen und diese beiden Außenschichten mit vollflächig verteilten Haltefasern durch die innere Schicht hindurch miteinander zu verbinden. Die beiden Schichten können dabei bahnförmig ausgebildet sein, wodurch die Herstellung kontinuierlich vorgenommen werden kann. Wird dabei mindestens für eine der beiden Außenschichten eine aktiv nadelfähige Schicht verwendet, so können die beiden Außenschichten durch die flächenförmig vorliegende innere Schicht hindurch miteinander vernadelt werden. Hierfür kann vorteilhaft eine aus der Nadelvliesteppichherstellung bekannte Nadelmaschine zur Anwendung gelangen. Gemäß einem weiteren Verfahrensschritt werden die drei miteinander durch Haltefasern verbundenen, bahnförmigen Schichten in anwendungsgemäße Flächengrößen aufgeteilt und eine so entstandene Wärmepackung zwischen zwei gas- und wasserundurchlässigen Folien verpackt, insbesondere vakuumverpackt. Wird dieses Aufteilen in anwendungsgemäße Flächengrößen mit heißen Werkzeugen vorgenommen und werden die drei Schichten gleichzeitig unmittelbar neben der Schneidelinie mechanisch aufeinanderzu gepreßt, so erhält man die obenbeschriebenen Wärmepackungen mit den gegenüber der Restfläche dünneren Randstreifen.

Gemäß einer besonderen Ausführungsform können die Haltefasern gleichmässig voneinander beabstandet über die gesamte Fläche der Wärmepackung verteilt sein.

Weitere Vorteile und Einzelheiten der Erfindung ergeben sich aus den im folgenden anhand der Zeichnung beschriebenen Ausführungsbeispielen.

Es zeigt :

Figur 1 Einen Teil einer Vorrichtung zur Herstellung einer Wärmepackung ;

Figur 2 Ein Teilstück einer Wärmepackung im Schnitt gemäss einer ersten Ausführungsform ;

Figur 3 Ein Teilstück einer Wärmepackung im Schnitt gemäss einer zweiten Ausführungsform, und

Figur 4 Einen Schnitt durch den Randbereich einer verpackten Wärmepackung gemäss einer dritten Ausführungsform.

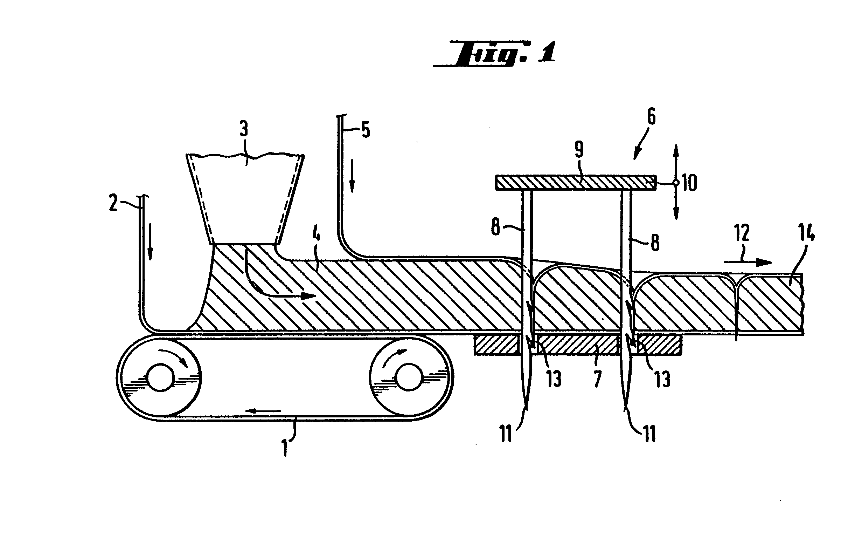

Vorab wird anhand der Fig. 1 der für die Herstellung der Wärmepackung wesentliche Schritt des Einbringens von Haltefasern, nämlich hier von durch Vernadeln eingebrachte Haltefasern beschrieben.

Gemäss Fig. 1 wird auf eine Fördereinrichtung, hier ein Förderband 1, eine erste Aussenschicht 2 abgelegt, auf die, hier von einer Austragsvorrichtung 3 dosiert, die innere Schicht 4 aus erdartiger Masse und Schmelzmasse aufgetragen wird. Auf diese Schicht 4 werden aktiv nadelfähige Fasern, hier in Form eines Faservlieses 5 aufgelegt, worauf dieses Drei-Schichten-System einer Nadelmaschine 6 zugeführt wird.

Solche Nadelmaschinen sind aus der textilen Nadelvliestechnik bekannt (vgl. z. B. Krcma, Textilverbundstoffe, Seite 139-141). Bei einer solchen Nadelmaschine 6 wird das zu vernadelnde System, hier das Drei-Schichten-System, über eine mit Bohrungen versehene Grundplatte 7 geführt. Oberhalb des zu vernadelnden Gegenstandes ist ein die Vernadelungsnadeln 8 tragendes Nadelbrett 9 angeordnet, welches sich fortwährend soweit auf und ab bewegt (Doppelpfeil 10), dass die Nadelspitzen 11 in ihrer untersten Stellung den zu vernadelnden Gegenstand gewöhnlich ganz durchdrungen haben, während sie in ihrer obersten Stellung mit dem noch zu vernadelnden Gegenstand keine Berührung aufweisen. In dieser obersten Stellung kann der zu vernadelnde Gegenstand, hier das Drei-Schichten-System, in Vorschubrichtung (Pfeil 12) taktweise verschoben werden, während es beim eigentlichen Vernadeln stillstehen muss. Die Vernadelungsnadeln 8 tragen an ihrem Schaft mindestens einen — hier zwei — Widerhaken 13 mit denen sie einzelne Fasern oder Faserbüschel ergreifen und in den zu vernadelnden Gegenstand hineinziehen, bzw. durch diesen hindurchziehen. Beim Zurückfahren der Nadeln 8 lösen sich die mitgenommenen Fasern oder Faserbüschel von den Widerhaken 13 und verbleiben in der passiv vernadelten Schicht, hier der unteren Aussenschicht 2 und der Gemischschicht 4. Während nun beim Vernadeln in der Textilindustrie, bei der Herstellung von Nadelvliesteppichen, die eine Enddicke von z. B. 4-6 mm aufweisen, die Nadelbretter 9 eine Vielzahl von dicht beieinander angeordneten Nadeln besitzen und dieses Nadelbrett z. B. mit einer Geschwindigkeit von 700 Hüben pro Minute bewegt werden kann, sollte beim Herstellen der für Wärmepackungen vorgesehenen Bahnen, die erdartige Masse und Paraffin zwischen den beiden Aussenschichten 2 und 5 enthalten, die Anzahl der Nadeln 8 in dem Nadelbrett 9 verkleinert und die Hubzahl, z. B. auf 200 bis 500 Hübe pro Minute, verringert werden.

Darüberhinaus empfiehlt es sich, sowohl die Nadeln 8, als auch die Grundplatte 7 und das Nadelbrett 9, z. B. mittels Heissluft, auf die Schmelztemperatur der Schmelzmasse zu erwärmen, damit die Nadeln leichter die innere Schicht durchdringen können. Auch empfiehlt sich, die innere Schicht in der Austragsvorrichtung 3 auf eine Temperatur oberhalb der Schmelztemperatur der Schmelzmasse zu erhitzen, so dass die innere Schicht 4 selbst noch zähflüssig bleibt. Stechen nun die Vernadelungsnadeln 8 durch das Drei-Schichten-System hindurch und werden dann wieder aus der oben aufliegenden Aussenschicht 5 herausgezogen, so löst sich die Schmelzmasse spätestens an der Aussenschicht 5 wieder von den Vernadelungsnadeln 8 ab.

Wie aus Fig. 1 ersichtlich, verringert sich beim Vernadeln des Drei-Schichten-Systems die Dicke desselben, da zum einen die Fasern enthaltende Aussenschicht 5 durch das Vernadeln verdichtet wird, zum anderen diese Aussenschicht 5 und, je nach Ausbildung, auch die andere Aussenschicht 2 in die Randbereiche der inneren Schicht 4 hineingezogen bzw. hineingedrückt werden.

Die aus der Nadelmaschine austretende Bahn 14 wird daraufhin in einzelne anwendungsgemässe Packungsgrössen zertrennt und zwischen zwei Folien 15 und 16 verpackt, worauf später noch im Zusammenhang mit der Fig. 4 näher eingegangen wird.

Fig. 2 zeigt nun eine vernadelte Wärmepackung, bei der zwischen den beiden — hier — aus je einem Faservlies bestehenden Aussenschichten 2 und 5 eine Schicht 4 mit Partikeln 17 aus erdartiger Masse und Paraffinpartikeln 18 angeordnet ist, in vergrösserter und schematisierter Darstellung, wobei ersichtlich ist, dass die beiden Aussenschichten 2 und 5 über die Haltefasern 19, die sich durch die Gemischschicht 4 hindurch erstrecken, verbunden sind. Die Wärmepackung ist hier von beiden Aussenschichten 2 und 5 her vernadelt, was durch die « Fasertrichter » 20 ersichtlich ist, die sich an den Einstichstellen der Vernadelungsnadeln 8 ausbilden. In diese Fasertrichter 20 werden auch Faserenden und Faserteile von Fasern, die nicht durch die Widerhaken 13 ergriffen werden, teilweise hineingezogen. Die die Heilpackung durchdringenden Haltefasern 19 sind über die Fläche der Bahn ungleichmässig verteilt, weshalb bei einem Schnitt durch eine solche Heilpackung in der Praxis nur sehr wenig Haltefasern 19 zu sehen sind.

Es ist nun ersichtlich, dass zwischen den Partikeln 17 aus erdartiger Masse, die z. B. aus Moor, Fango, Lehm (Heilerde) bestehen kann, und den Paraffinpartikeln 18 noch Zwischenräume verbleiben, die jedoch in der Praxis kleiner sind, als hier in der schematisierten Darstellung, da, wie oben gesagt, durch das Vernadeln der drei Schichten die beiden Aussenschichten nach dem Vernadeln näher beieinander liegen, als vor dem Vernadeln. Dies ist zum Einen eine Folge des Vernadelungsvorgangs als Solchen, bei dem sehr starke Vibrationen auftreten, andererseits ziehen die Haltefasern 19 die beiden Aussenschichten 2 und 5 aufeinanderzu, so dass diese einen gewissen Druck auf die Gemischschicht 4 ausüben.

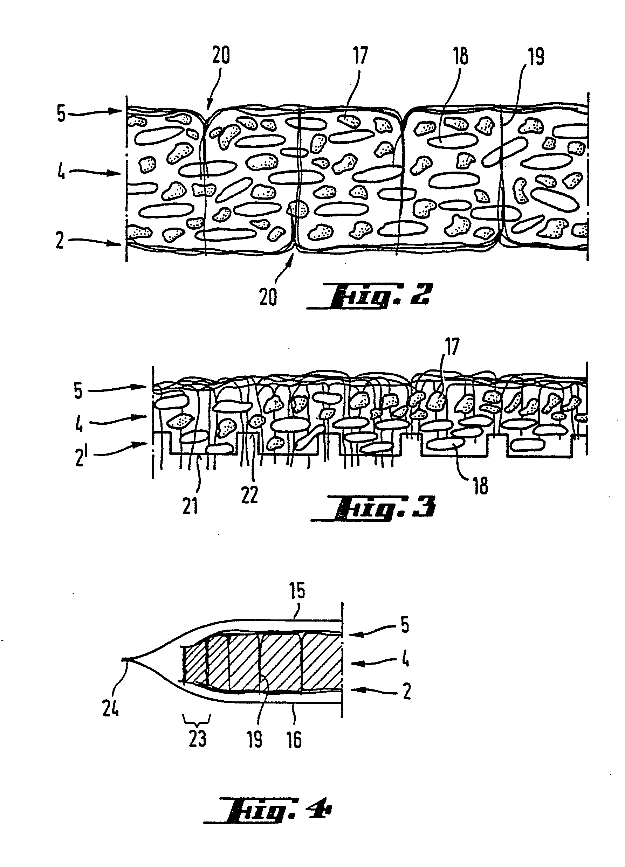

Fig. 3 zeigt nun eine wesentlich andere Ausführungsform, da dort die eine der beiden Aussenschichten, nämlich die in der Zeichnung untere, durch eine sogenannte Näpfchenfolie 2' gebildet wird.

Diese Näpfchenfolie 2' kann eine Kunststofffolie, eine auf eine Kunststofffolie kaschierte Alufolie, eine Alufolie oder auch ein Faserverbundstoff sein, wobei die Näpfchen 21 z. B. durch Tiefziehen, insbesondere im warm plastischen Zustand, erzielt werden können. Die Vertiefungen dieser Näpfchen 21 sind mit dem Gemisch — oder zumindest mit einem Bestandteil des Gemisches — gefüllt, wobei die Gesamtdicke der Gemischschicht 4 grösser ist, als die Tiefe der Näpfchen 21. In Fig. 3 sind nun die Haltefasern ganz gleichmässig über die Fläche der Heilpackung verteilt. Während im linken Teil der Fig. 3 die Haltefasern 19 durch die gesamte Wärmepackung hindurchdringen, d. h. sowohl durch den Boden der Näpfchen 21, als auch durch die die Näpfchen verbindenden Stege 22, sind im rechten Teil der Fig. 3 die Haltefasern nicht so tief eingenadelt, so dass sie die Folie 2' nur im Bereich der Stege 22 durchdringen, im Boden der Näpfchen 21 jedoch nicht gehalten werden. Eine Ausbildung gemäss dem rechten Teil der Fig. 3, bei dem die Haltefasern nur die Stege durchdringen, weist deshalb eine dichtere Aussenschicht 2' auf, als eine Packung entsprechend dem linken Teil der Fig. 3.

Der linke und der rechte Teil der Fig. 3 unterscheiden sich nun aber auch noch darin, dass im linken Teil die Partikel aus erdförmiger Masse 17 und die Paraffinpartikel 18 über die gesamte Dicke der Packung gleichmässig verteilt sind, während im Bereich der beiden rechten Näpfchen 21 die Partikel in Schichten getrennt vorliegen, wobei benachbart der Folie 2' im wesentlichen nur Paraffinpartikel 18 vorliegen, während benachbart der durchlässigeren Faservliesschicht 5 im wesentlichen die Partikel aus erdartiger Masse 17 vorhanden sind. Wird eine solche Wärmepackung über den Schmelzpunkt des Paraffins erhitzt, so dass das Paraffin flüssig wird, bleibt es im wesentlichen unvermischt mit den Partikeln 17 aus erdartiger Masse, deren Wirkung sich auch dann noch entfalten kann.

Fig. 4 zeigt nun eine Wärmepackung, die zwischen zwei Folien 15 und 16 verpackt ist, im Teilschnitt. Die Wärmepackung weist an ihren Rändern 23 eine geringere Dicke auf, als beabstandet von diesen Rändern 23, die dadurch erhalten wird, dass beim Schneiden der vernadelten Bahn 14, welches z. B. mittels eines heissen Messers vorgenommen werden kann, unmittelbar neben diesen Schneidelinien die beiden Aussenschichten 2 und 5 mechanisch aufeinanderzu gepresst werden, wobei durch diesen Pressdruck die dort vorliegenden Fasern mit der Schmelzmasse verklebt werden. Die Ränder 23 der Heilpackung werden dadurch quasi-versiegelt.

Diese Ausführungsform zeigt nun eine innere Schicht 4 der Wärmepackung, bei der die Schmelzmasse mit der erdartigen Masse vollständig vermischt vorliegt, d. h. bei der Herstellung wurde eine Schmelze aus allen Bestandteilen der inneren Schicht als solche aufgegeben. Die innere Schicht 4 ist in sich homogen und wird nur von den Haltefasern 19 durchdrungen.

Gemäss einer nicht dargestellten Ausführungsform kann eine zuvor beschriebene Wärmepackung auch noch an ihren Aussenseiten, an denen die anderen Wärmepackungen Fasern aufweisen, mit einer dünnen Schicht aus erdartiger Masse und Paraffin beschichtet sein, so dass die Fasern der Aussenschicht bei der fertigen Packung nicht sichtbar sind.

Eine solche konfektionierte Wärmepackung wird dann zwischen die beiden einen offenen Beutel bildenden Folien 15 und 16 eingeschoben, worauf der offene Beutel unter Absaugen der in ihm enthaltenen Luft verschlossen wird. Durch das Vorliegen eines Vakuums in dem Folienbeutel 15 und 16 wird eine gute Haltbarkeit über eine längere Lagerdauer gewährleistet. Ausserdem kann die in diesem Beutel verpackte Wärmepackung in einen Behälter mit kochendem Wasser, z. B. in einen Kochtopf gegeben werden, wobei die Wärmepackung selbst trocken bleibt und das Paraffin zum Schmelzen gebracht werden kann. Soll die Wärmepackung dagegen feucht benutzt werden, so können z. B. mit einer Gabel kleine Öffnungen in der Verpackung angebracht werden, ohne dass die Wärmepackung selbst ungeschützt in das Wasser gelegt werden muss.

Die Zusammensetzung und der Aufbau einiger erfindungsgemässer Wärmepackungen ergibt sich aus folgenden Beispielen.

Beispiel 1

Zur Herstellung der einen, mindestens Flüssigkeit durchlassenden Aussenschicht 5 wurde ein Vlies aus lebensmittelechten Polypropylenfasern mit einem Flächengewicht von 90 g, einem Titer von 6,7 dtex und einer Stapellänge von 90 mm auf einen Bafatexträger mit einem Flächengewicht von 25 g pro m² abgelegt. Dieses Vlies wurde mit dem passiv nadelfähigen Bafaexträger mit einer Stichdichte von 48 Stichen pro cm² vorvernadelt.

Für die andere Aussenschicht 2 wurde ein Faservlies mit den gleichen Fasern verwendet, wobei dieses Faservlies ein Flächengewicht von 150 g pro cm² aufwies. Auch dieses Faservlies wurde auf einen Bafaexträger aufgenadelt, wobei diese Vorvernadelung mit einer Stichdichte von 72 Stichen pro cm² durchgeführt wurde. Diese Aussenschicht 2 war dadurch dichter, wie die zuvor beschriebene Aussenschicht 5.

Für die Gemischschicht 4 wurde ein Gemisch aus vier Gewichtsteilen getrocknetem Moor und sechs Gewichtsteilen geraspelten Paraffins verwendet. Dieses Gemisch wurde mit einem Flächengewicht von 12 kg pro m² auf die schwerere Aussenschicht 2 aufgelegt, wie dies anhand der Fig. 1 oben beschrieben wurde, worauf diese Gemischschicht mit der leichteren Aussenschicht 5 abgedeckt und das Drei-Schichten-System von beiden Seiten her vernadelt wurde. Die Stichdichte jeder Seite betrug dabei 24 Stiche pro cm². Die vernadelte Bahn 14 wurde dann in einzelne Wärmepackungen, die eine Fläche von 20 × 15 bis 30 × 20 cm aufwiesen geschnitten, wobei die beiden Aussenschichten 2 und 5 an dem umlaufenden Rand aufeinanderzu gepresst wurden. Man erhielt eine Wärmepackung, deren beiden Oberflächen aus Fasern bestanden, wobei die eine Aussenschicht 5 durchlässiger war als die andere Aussenschicht 2 und Erstere auf die zu behandelnden Körperteile aufgelegt werden soll. Die Wärmepackungen wurden dann zwischen die Beutel bildenden Folien gelegt, die in dem Beutel befindliche Luft weitgehend abgesaugt und die Beutelöffnung verschweisst.

Zur Anwendung wurde der geschlossene Beutel in einen Kochtopf mit kochendem Wasser geworfen und diesem nach 20 Minuten wieder entnommen. Nach Aufreissen des Beutels und Entnahme der Wärmepackung konnte diese mit ihrer durchlässigeren Aussenschicht 5 auf zu behandelnde Körperstellen gelegt werden.

Beispiel 2

Die beiden Aussenschichten 2 und 5 waren hier genauso aufgebaut, wie die beiden Aussenschichten 2 und 5 des Beispieles 1 mit Ausnahme, dass hier jeweils ein Polypropylenfasergemisch verwendet wurde, wobei das Gemisch 85 % Fasern eines Titers von 6,7 dtex und 15 % Fasern eines Titers von 17 dtex aufwies.

Als Gemischschicht 4 wurde ein Gemisch aus einem Gewichtsteil Fango und einem Gewichtsteil geraspelten Paraffinkörnern verwendet, wobei dieses Gemisch mit einem Flächengewicht von 10 kg pro m² zwischen die beiden Aussenschichten 2 und 5 gebracht wurde, die genauso wie im Beispiel 1 vernadelt und verpackt wurden. Vor der Anwendung wurde nun diese Parafango-Wärmepackung aus dem Beutel genommen und in einem mit Heilkräutern angesetztem Wasserbad, welches auf einer normalen Kochplatte so erhitzt wurde, dass sich lediglich am Boden des Topfes Dampfblasen bildeten, 10 Minuten lang erwärmt.

Bei der Anwendung dieser Wärmepackung auf dem menschlichen Körper kamen im wesentlichen die von der Wärmepackung aufgenommenen Kräuterbestandteile zur Wirkung.

Beispiel 3

Als durchlässigere Aussenschicht 5 wurde hier wieder die oben beschriebene leichtere Faserschicht verwendet. Als dichtere Aussenschicht 2' wurde eine Näpfchenfolie verwendet, d. h. eine aluminiumbeschichtete Kunststoffolie, in der durch Tiefziehen kreisförmige Vertiefungen mit einem Durchmesser von 10 mm und einer Tiefe von 5 mm vorgesehen waren, wobei die Aluminiumschicht sich auf der Seite befand, auf der durch die Näpfchen die Vertiefungen ausgebildet werden. Als Kunststoffolie wurde dabei eine Polyäthylenfolie mit einem Flächengewicht von 115 g pro m² verwendet. Auf die Aluminiumschicht, insbesondere in die Vertiefungen hinein, wurde eine Schicht von fein geraspeltem Paraffin mit einem Flächengewicht von 7 kg pro m² abgelegt. Diese Paraffinschicht wurde mit einer Schicht aus getrocknetem Moor mit einem Flächengewicht von 3 kg pro m² bedeckt, woraufhin diese Moorschicht mit der Faserschicht abgedeckt wurde. Das so gebildete Mehrschichtensystem wurde nun von der Faserschicht her mit einer Stichdichte von 72 Stichen pro cm² vernadelt, wobei die Fasern nicht nur die zwischen den Vertiefungen befindlichen Stege, sondern auch den Boden der Vertiefungen durchdrungen haben.

Diese Wärmepackung wurde wieder in den Folienbeutel, wie im Beispiel 1 beschrieben, verpackt und dann in einem heissen, dampfenden Wasserbad 20 Minuten erwärmt. Der Beutel wurde dem Wasserbad entnommen, geöffnet und für etwa eine Minute so über den Wasserdampf gehalten, dass dieser auf die Faserschicht mit der dahinter liegenden Moorschicht gelangt. Es konnte dabei mittels eines Parallelmusters festgestellt werden, dass auch nach dem Erhitzen keine wesentliche Vermischung des Paraffins mit dem Moor eingetreten war.

Die so erhitzte und befeuchtete Packung wurde wieder mit der Faserschicht auf den zu behandelnden Körperteil aufgelegt. Nach 15 Minuten wurde durch die immer noch aufliegende Wärmepackung hindurch der Körperteil massiert. Es konnte dabei festgestellt werden, dass keine wesentliche Verschiebung der zwischen den beiden Aussenschichten liegenden Masse aus Moor und Paraffin eintritt, während jedoch kleinere Moorpartikel insbesondere durch die ausgeübte Massage durch die Faserschicht hindurch getreten waren. Weiterhin konnte beobachtet werden, dass durch die Kunststoffolie an den durch die Haltefasern perforierten Stellen im wesentlichen trotz der Massage kein Paraffin ausgetreten war, sondern das Paraffin praktisch die perforierten Stellen restlos verschloss.

Beispiel 4

Es wurde dasselbe Muster, wie in Beispiel 3 nachgebildet, wobei nun hier die Haltefasern nur so tief eingenadelt wurden, dass sie lediglich die zwischen den Vertiefungen befindlichen Stege durchdrangen, vom Boden der Vertiefung jedoch beabstandet waren. Um diesen Boden auch nicht durch die Nadelspitzen zu perforieren, wurden spezielle aus der Textiltechnik bekannte Nadeln verwendet, bei denen der Abstand des zur Nadelspitze hin letzten Widerhakens zu dieser Nadelspitze sehr gering ist.

Obwohl hier nur ein Teil der Haltefasern beide Aussenschichten 2 und 5 miteinander verband, konnte auch durch diese Wärmepackung hindurch massiert werden, da die die beiden Aussenschichten noch im Bereich der Stege verbindenden Haltefasern ausreichend waren, um die Moorpartikel bzw. das Paraffin an einem Verschieben quer zur Flächenerstreckung der Schichten zu hindern.

Beispiel 5

Zur Herstellung der beiden Aussenschichten 2 und 5 wurde je ein Vlies aus lebensmittelechten Polypropylenfasern mit einem Flächengewicht von 120 g pro m², einem Titer von 17 dtex und einer Stapellänge von 90 mm auf einen grossmaschigen Träger aus sich kreuzenden Fäden mit einem Flächengewicht von 42 g pro m² abgelegt. Dieses Vlies wurde mit dem passiv nadelfähigen Träger mit einer Stichdichte von 27 Stichen pro cm² vernadelt, sodass sich vorvernadelte Aussenschichten mit einem Gewicht von 162 g pro m² ergaben. Für eine homogene innere Schicht 4 wurden 45 Gewichtsprozent Paraffin, 43,5 Gewichtsprozent Fangopulver, 5 Gewichtsprozent Talk, und 5,5 Gewichtsprozent Verdicker und Mittel zur Verhinderung des Blutens nach dem Schmelzen des Paraffins solange gemischt, bis sich eine gleichmässige Mischung ergab. Diese noch flüssige Mischung wurde mittels der Austragsvorrichtung auf die Aussenschicht 2 mit einer Schichtdicke von 12 mm und einem Gewicht von 16 kg pro m² aufgetragen. Auf diese Gemischschicht wurde die zweite Aussenschicht 5 aufgelegt. Dieses Drei-Schichten-System wurde von beiden Aussenschichten 2 und 5 her vernadelt, wobei die Stichdichte in jeder Seite 24 Einstiche pro cm² betrug. Während des Vernadelns lag der grösste Teil des Gemisches noch im geschmolzenen Zustand vor, nur umhüllt von schon erstarrten Aussenbereichen des Gemisches und von den beiden Aussenschichten. Die Nadeln 8, das Nadelbrett 9 und die Grundplatte 7 wurden mit Heissluft etwa auf die Schmelztemperatur der Schmelzmasse erhitzt, so dass die in die innere Schicht 4 eingedrungenen Nadeln beim Herausziehen an der Faserschicht 5 jeweils wieder von der Schmelzmasse befreit wurden.

Die vernadelte Bahn 14 wurde dann in einzelne Wärmepackungen, die eine Fläche von 15 × 40 cm aufwiesen, geschnitten. Man erhielt dabei eine Wärmepackung, deren beiden Oberflächen aus Fasern bestanden. Einzelne Wärmepackungen wurden dann zwischen die Beutel bildenden Folien 15 und 16 gelegt, die in dem Beutel befindliche Luft wurde abgesaugt und die Beutelöffnung verschweist.

Zur Anwendung wurde der geschlossene Beutel mit der Wärmepackung in einen Topf mit siedendem Wasser gelegt, welches weiter kochte, und dort 15 Minuten belassen. Danach wurde die Packung aus dem Beutel herausgenommen und nach ein paar Minuten der Abkühlung die Oberflächentemperatur der Packung geprüft. Dieses Prüfen wurde einfach dadurch vorgenommen, dass der Handrücken auf die Packung aufgelegt wurde, um festzustellen, ob die Haut die Wärme erträgt. In diesem Falle hatte die Packung etwa eine Temperatur von 52-54 °C.

Diese streifenförmige Packung wurde dann auf die zu behandelnde Stelle des Körpers aufgelegt, insbesondere bei Armen und Beinen um diese Gliedmassen gewickelt. Die Packung liess sich sehr leicht anmodelieren, so dass sie vollflächig auf den betreffenden Körperteilen auflag. Mit einer vorbereiteten Haushaltsfolie und einer Wolldecke wurde die Packung abgedeckt, bevor der Patient zur Ruhe gebettet wurde.

Nach der Behandlung, die zwischen 20 und 45 Minuten betrug, wurde die Packung abgenommen, flach ausgelegt in den Beutel zurückgegeben, das ganze glatt gestrichen, um die Luft aus dem Beutel möglichst weitgehend zu entfernen, und die Beutelöffnung wieder verschlossen.

Eine solche Wärmepackung kann mehrere Male wiederverwendet werden, so dass es für eine Behandlung, die sich über mehrere Wochen hinzieht, ausreicht, eine einzige Wärmepackung zu benutzen. Während der Lagerung soll die verpackte Wärmepackung möglichst kühl und trocken gelagert werden, vor der nächsten Benutzung wird sie wieder in ein kochendes Wasserbad gegeben, in dem die Packung wieder weich und elastisch wird.

Wenngleich die Packung eine Länge von 40 cm aufweist, ist es nicht notwendig, ein Wasserbad zu benutzen, in dem die steife Packung voll Platz hat, da beobachtet werden konnte, dass der zuerst in das Wasserbad eingetauchte Teil der Packung sehr schnell so weich wird, dass der Rest der Packung in das Wasserbad nachrutschen kann. Der hierbei auftretende Effekt ist ähnlich wie beim Kochen von Spaghetti, bei denen auch zuerst der im Wasser befindliche Teil so weich wird, dass danach der andere Teil nachrutschen kann.

Insbesondere durch die zuvor beschriebenen Beispiele wird deutlich, dass eine erfindungsgemäss ausgebildete Wärmepackung vielseitiger verwendet werden kann, als die bekannten Wärmepackungen, so kann durch die dünnere Aussenschicht 5 die dahinterliegende Gemischschicht bzw. die unmittelbar dahinterliegende Schicht aus erdiger Masse, da diese im wesentlichen trocken ist, auch mit Ölen, Schwefel, Pflanzenextrakten oder dergleichen getränkt werden. Dieses Tränken kann auch unmittelbar vor der Anwendung, insbesondere aber auch von fachlich nicht vorgebildeten Personen durchgeführt werden.

Soll bei bestimmten Heilbehandlungen die Wärme länger auf den zu behandelnden Körperteil einwirken, so ist es möglich, die erfindungsgemässe Wärmepackung so zu falten, dass verschiedene Abschnitte der Wärmepackung übereinander zu liegen kommen. Es ergibt sich somit eine Wärmepackung, die zwar nurmehr z. B. die halbe Fläche aufweist, deren Schichtdicke jedoch dann doppelt so gross ist. Die Wärmepackungen können auch schon gefaltet geliefert werden, wodurch beim Erwärmen in dem wassergefüllten Topf weniger Platz benötigt wird.

Beispiel 6

Auf eine aluminiumbeschichtete Kunststoff-Folie 2', in der durch Tiefziehen kreisförmige Vertiefungen mit einem Durchmesser von 20 mm und einer Tiefe von 10 mm vorgesehen waren, wurde ein erhitztes Gemisch aus 75 Gewichtsprozent Paraffin, 10 Gewichtsprozent Ceresin, 10 Gewichtsprozent Sandpulver und 5 Gewichtsprozent Talk aufgetragen und mit einer Faserschicht abgedeckt. Als Kunststoff-Folie wurde dabei eine Polyäthylenfolie mit einem Flächengewicht von 115 g pro m² verwendet. Die Gemischschicht 4 hatte ein Flächengewicht von 20 kg pro m², während die Faserschicht ein Flächengewicht von 120 g pro m² aufwies, und auf einem grossmaschigen Träger vorvernadelt war. Diese drei Schichten wurden nun so vernadelt, dass die Haltefasern nur zwischen den Vertiefungen durch die Folie hindurch drangen und in den Vertiefungen selbst keine Nadeleinstiche waren.

Ein solches Drei-Schichten-System wurde in Bahnen von 15 cm Breite und jeweils einer Länge von 10 m hergestellt und aufgerollt. Diese, eine andere Art von Wärmepackungen bildende Bahnen wurden in Gärtnereien zwischen Reihen von klein-wüchsigen Pflanzen so ausgerollt, dass die Kunststoff-Folie auf dem Erdboden zu liegen kam, während die Faserschicht dem Sonnenlicht ausgesetzt war. Während des Tages erwärmte auch geringere Sonneneinstrahlung die Wärmepackung, was je nach Intensität und Dauer der Sonneneinstrahlung bis zum Schmelzen des Paraffin-Ceresin-Gemisches führte. Während der Nacht gab diese Packung ihre Wärme langsam an die benachbarten Pflanzen und die benachbarte Erdbodenschicht ab, so dass sowohl die Pflanzen, als auch der Erdboden auf einer Temperatur gehalten werden konnten, die verglichen mit Bereichen, in denen keine Wärmepackungen verwendet wurden, um einige °C höher war. Bei dieser Anwendung dient die Wärmepackung im wesentlichen als Wärmespeicher, der die am Tag aufgenommene Wärme bei Nacht wieder abgibt.

Eine erfindungsgemässe Wärmepackung kann auch eine eigene Heizeinrichtung enthalten, wobei z. B. zwischen den beiden Aussenschichten neben der erdartigen Masse und der Schmelzmasse noch isolierte Heizdrähte eingebracht sind. Insbesondere beim Vernadeln eines solchen Drei-Schichten-Systems ist dann darauf zu achten, dass die Nadeln neben den Heizdrähten einstechen, so dass deren Isolation nicht beschädigt wird. Andererseits kann aufgrund der speziellen Ausbildung der Wärmepackung diese auch mittig gefaltet werden, wodurch zwei Packungsteile aufeinander liegen. Zwischen diesen beiden Packungsteilen können dann die Heizdrähte angeordnet sein. Aufgrund des grossen Wärmespeichervermögens der Packung kann diese Wärmepackung ihre Wärme auch noch lange Zeit nach Abschalten des elektrischen Stromes bzw. insbesondere nach Herausziehen des Steckers der elektrischen Leitung Wärme abgeben.

**Patentansprüche**

1. Wärmepackung, bei der eine erdartige Masse zwischen zwei die Masse umhüllenden, flächigen Außenschichten (2, 5) gelagert ist und beide Außenschichten (2, 5) mindestens an ihrem Rand miteinander verbunden sind, dadurch gekennzeichnet, daß zwischen den beiden Außenschichten (2, 5) eine Schicht (4) aus der erdartigen Masse und einer Schmelzmasse aus synthetischen und/oder natürlichen Kohlenwasserstoffwachsen vorliegt und die drei Schichten (2, 4, 5) durch vollflächig verteilte Haltefasern (19) durch die innere Schicht (4) hindurch miteinander verbunden sind.

2. Wärmepackung nach Anspruch 1, dadurch gekennzeichnet, daß die drei Schichten (2, 4, 5) miteinander vernadelt sind.

3. Wärmepackung nach Anspruch 1 oder 2, dadurch gekennzeichnet, daß als erdartige Masse Moor, Fango, Lehm (Heilerde) oder dergleichen, insbesondere in im wesentlichen getrockneter Form vorliegt.

4. Wärmepackung nach einem der vorhergehenden Ansprüche, dadurch gekennzeichnet, daß als Schmelzmasse Paraffin vorliegt.

5. Wärmepackung nach einem der vorhergehenden Ansprüche, dadurch gekennzeichnet, daß die zwischen den beiden Außenschichten (2, 5) liegende Schicht (4) aus 30-70 Gewichtsprozent erdartiger Masse und aus 70-30 Gewichtsprozent Schmelzmasse besteht, vorzugsweise ein Mischungsverhältnis von 1 : 1 vorliegt.

6. Wärmepackung nach einem der vorhergehenden Ansprüche, dadurch gekennzeichnet, daß die Schicht (4) aus erdartiger Masse und Schmelzmasse zwischen den beiden Außenschichten (2, 5) mit einem Flächengewicht von 3-30 kg pro m², vorzugsweise 8-15 kg pro m² vorliegt.

7. Wärmepackung nach einem der vorhergehenden Ansprüche, dadurch gekennzeichnet, daß der Schicht (4) aus erdartiger Masse und Schmelzmasse Wirkstoffe, wie Heilkräuter, Öle, Schwefel, Pflanzenextrakte oder dergleichen, beigemischt sind.

8. Wärmepackung nach einem der vorhergehenden Ansprüche, dadurch gekennzeichnet, daß mindestens die eine Außenschicht (5) aus einem aktiv nadelfähigen Faservlies besteht.

9. Wärmepackung nach dem vorhergehenden Anspruch, dadurch gekennzeichnet, daß dieses Faservlies (5) ein Flächengewicht von 20-350 g pro m², vorzugsweise von 100-180 g pro m² aufweist.

10. Wärmepackung nach einem der beiden vorhergehenden Ansprüche, dadurch gekennzeichnet, daß auch die andere Außenschicht (2) aus einem Faservlies besteht und vorzugsweise ein Flächengewicht von 100-180 g pro m² aufweist.

11. Wärmepackung nach einem der Ansprüche 1-9, dadurch gekennzeichnet, daß die zweite Außenschicht (2') aus einer Folie besteht und vorzugsweise ein Flächengewicht von 50-200 g pro m² aufweist.

12. Wärmepackung nach dem vorhergehenden Anspruch, dadurch gekennzeichnet, daß diese Folie (2') eine Aluminiumfolie ist.

13. Wärmepackung nach einem der beiden vorhergehenden Ansprüche, dadurch gekennzeichnet, daß diese Folie (2') eine Näpfchenfolie ist.

14. Wärmepackung nach einem der vorhergehenden Ansprüche, dadurch gekennzeichnet, daß in der Nähe einer mindestens für Flüssigkeiten durchlässigen Außenschicht (5) der Anteil der derdartigen Masse (17) größer ist, als in der Nähe der anderen Außenschicht (2).

15. Wärmepackung nach einem der vorhergehenden Ansprüche, dadurch gekennzeichnet, daß die Haltefasern (19) in einer solchen Flächendichte vorgesehen sind, daß sie die erdartige Masse und die Schmelzmasse an einem Verschieben hindern.

16. Wärmepackung nach einem der vorhergehenden Ansprüche, dadurch gekennzeichnet, daß die Haltefasern (19) die beiden Außenschichten (2, 5) aufeinanderzu ziehen, so daß diese auf die Schicht (4) aus erdartiger Masse und Schmelzmasse einen Druck ausüben.

17. Wärmepackung nach einem der vorhergehenden Ansprüche, dadurch gekennzeichnet, daß das Drei-Schichten-System (2, 4, 5) an seinen umlaufenden Rändern (23) eine geringere Dicke aufweist, als beabstandet von diesen Rändern (23).

18. Wärmepackung nach einem der vorhergehenden Ansprüche, dadurch gekennzeichnet, daß die erdartige Masse in körniger Form vorliegt.

19. Wärmepackung nach einem der vorhergehenden Ansprüche, dadurch gekennzeichnet, daß die erdartige Masse in im wesentlichen getrockneter Form vorliegt.

20. Wärmepackung nach einem der vorhergehenden Ansprüche, dadurch gekennzeichnet, daß die Schmelzmasse in körniger Form vorliegt.

21. Wärmepackung nach einem der Ansprüche 1-19, dadurch gekennzeichnet, daß die Schmelzmasse als durchgehende, von den Haltefasern durchdrungene Schicht vorliegt.

22. Wärmepackung nach dem vorhergehenden Anspruch, dadurch gekennzeichnet, daß die erdartige Masse in die Schmelzmasse eingebettet ist.

23. Wärmepackung nach einem der vorhergehenden Ansprüche, dadurch gekennzeichnet, daß die aus den beiden Außenschichten (2, 5) und der Schicht (4) aus erdartiger Masse und Schmelzmasse gebildete Packung zwischen zwei gas- und flüssigkeitsdichten Folien (15, 16) angeordnet ist und diese Folien (15, 16) an ihren Rändern (24) abgedichtet miteinander verbunden sind.

24. Wärmepackung nach dem vorhergehenden Anspruch, dadurch gekennzeichnet, daß zwischen diesen beiden Folien (15, 16) ein Vakuum vorliegt.

25. Verfahren zur Herstellung einer Wärmepackung, bei der eine erdartige Masse zwischen zwei die Masse umhüllenden, flächigen Außenschichten (2, 5) gelagert ist und beide Außenschichten (2, 5) mindestens an ihrem Rand miteinander verbunden sind, dadurch gekennzeichnet, daß eine Schicht (4) aus erdartiger Masse und Schmelzmasse aus synthetischen und/oder natürlichen Kohlenwasserstoffwachsen zwischen die beiden Außenschichten (2, 5) eingebracht wird und die beiden die innere Schicht (4) umgebenden Schichten (2, 5) mit vollflächig verteilten Haltefasern (19) durch die innere Schicht (4) hindurch miteinander verbunden werden.

26. Verfahren nach dem vorhergehenden Anspruch, dadurch gekennzeichnet, daß mindestens für eine der beiden Außenschichten eine aktiv nadelfähige Schicht verwendet wird und daß diese beiden Außenschichten durch die flächenförmig vorliegende innere Schicht hindurch miteinander vernadelt werden.

27. Verfahren nach einem der beiden vorhergehenden Ansprüche, dadurch gekennzeichnet, daß die drei miteinander durch Haltefasern verbundenen Schichten in anwendungsgemäße Flächengrößen aufgeteilt werden und eine so entstandene Packung zwischen zwei gas- und wasserundurchlässigen Folien verpackt wird.

28. Verfahren nach dem vorhergehenden Anspruch, dadurch gekennzeichnet, daß die Aufteilung in anwendungsgemäße Flächengrößen mit heißen Werkzeugen vorgenommen wird und die drei Schichten gleichzeitig unmittelbar neben der Schneidelinie mechanisch aufeinanderzu gepreßt werden.

29. Verfahren nach einem der beiden vorhergehenden Ansprüche, dadurch gekennzeichnet, daß die Packung zwischen den beiden Folien vakuumverpackt wird.

**Claims**

1. Heating pad in which an earthy mass is located between two flat outer layers (2, 5) enclosing the mass and the two outer layers (2, 5) are connected together at least at their edge, characterised in that between the two outer layers (2, 5) there is a layer (4) made of the earthy mass and a molten mass of synthetic and/or natural hydrocarbon wax and the three layers (2, 4, 5) are connected together by holding fibers (19) which pass through the inner layer (4) and are distributed all over the surface.

2. Heating pad according to claim 1 characterised in that the three layers (2, 4, 5) are needled together.

3. Heating pad according to claim 1 or 2 characterised in that peat, fango, loam (healing earth) or the like is present as the earthy mass, in particular in a substantially dried form.

4. Heating pad according to one of the foregoing claims characterised in that a paraffin material is present as the molten mass.

5. Heating pad according to one of the foregoing claims characterised in that the layer (4) which lies between the two outer layers (2, 5) is made up of 30-70 percent by weight of the earthy mass and 70-30 percent by weight of the molten mass, preferably a mixture of 1 : 1 ratio.

6. Heating pad according to one of the foregoing claims characterised in that the layer (4) of earthy mass and molten mass between the two outer layers (2, 5) has a weight per unit area of 3-30 kg per m$^2$, preferably 8-15 kg per m$^2$.

7. Heating pad according to one of the foregoing claims characterised in that the layer (4) of earthy mass and molten mass has mixed with it materials such as medicinal herbs, oils, sulphur, plant extracts or the like.

8. Heating pad according to one of the foregoing claims characterised in that at least the one outer layer (5) comprises a fibre fleece capable of being actively needled.

9. Heating pad according to the preceding claim characterised in that this fibre fleece (5) has a weight per unit area of 20-350 g per m$^2$, preferably 100-180 g per m$^2$.

10. Heating pad according to one of the two preceding claims characterised in that the other outer layer (2) as well is made of a fibre fleece and preferably has a weight per unit area of 100-180 g per m$^2$.

11. Heating pad according to one of claims 1-9 characterised in that the second outer layer (2') comprises a foil or film and preferably has a weight per unit area of 50-200 g per m$^2$.

12. Heating pad according to the preceding claim characterised in that this film or foil (2') is an aluminium foil.

13. Heating pad according to one of the two preceding claims characterised in that this foil (2') is a stepped foil.

14. Heating pad according to one of the foregoing claims characterised in that in the neighbourhood of an outer layer (5) which is permeable at least to liquids the proportion of earthy mass (17) is greater than in the neighbourhood of the other outer layer (2).

15. Heating pad according to one of the foregoing claims characterised in that the holding fibers (19) are provided in such density per unit area that they prevent displacement of the earthy mass and the molten mass.

16. Heating pad according to one of the foregoing claims characterised in that the holding fibers (19) draw the two outer layers (2, 5) together so that these layers exert pressure on the layer (4) which comprises the earthy mass and the molten mass.

17. Heating pad according to one of the foregoing claims characterised in that the three-layer system (2, 4, 5) has a smaller thickness at its peripheral edges (23) than in regions spaced away from these edges (23).

18. Heating pad according to one of the foregoing claims characterised in that the earthy mass is in granular form.

19. Heating pad according to one of the foregoing claims characterised in that the earthy mass is present in a substantially dried form.

20. Heating pad according to one of the foregoing claims characterised in that the molten mass is in granular form.

21. Heating pad according to one of claims 1-19 characterised in that the molten mass is present as a continous layer and through which the retaining threads penetrate.

22. Heating pad according to the preceding claim characterised in that the earthy mass is embedded in the molten mass.

23. Heating pad according to one of the foregoing claims characterised in that the package comprising the two outer layers (2, 5) and the layer (4) of earthy mass and molten mass is arranged between two gas-tight liquid-tight foils (15, 16) and these foils (15, 16) are bonded together at their edges (24).

24. Heating pad according to the preceding claim characterised in that a vacuum is present between these two foils (15, 16).

25. Method of manufacturing a heating pad in which an earthy mass is laid between two flat outer layers (2, 5) enclosing the mass and the two outer layers (2, 5) are joined together at least at their edge, characterised in that a layer (4) of earthy mass and molten mass of synthetic and/or natural hydrocarbon waxes is mounted in between the two outer layers (2, 5) and the two layers (2, 5) which enclose the inner layer (4) are connected together through the inner layer (4) by holding fibers (18) distributed all over the surface.

26. Method according to the preceding claim characterised in that a layer which is capable of being actively needled is used for at least one of the two outer layers and that these two outer layers are needled together through the inner layer present in flat form.

27. Method according to one of the two preceding claims characterised in that the three layers connected together by holding fibers are cut up into surface areas according to use and a package thus produced is packed between two gas-tight and water-tight foils.

28. Method according to the preceding claim characterised in that the cutting up into surface areas according to use is performed by hot tools and the three layers are simultaneously pressed together mechanically directly adjacent to the cutting line.

29. Method according to one of the two preceding claims characterised in that the package is vacuum-packed between the two foils.

**Revendications**

1. Poche chauffante, dans laquelle une masse de la nature de la terre est disposée entre deux couches extérieures planes (2, 5) enrobant la masse et où lesdites deux couches extérieures (2, 5) sont reliées l'une à l'autre au moins par leur bord, caractérisée en ce qu'une couche (4) composée de la masse de la nature de la terre et d'une masse à fondre constituée de cires d'hydrocarbures synthétiques et/ou naturelles est présente entre les deux couches extérieures (2, 5) et en ce que les trois couches (2, 4, 5) sont reliées les unes aux autres par des fibres de retenue (19) réparties sur toute la surface et passant par la couche intérieure (4).

2. Poche chauffante selon la revendication 1, caractérisée en ce que les trois couches (2, 4, 5) sont aiguilletées entre elles.

3. Poche chauffante selon l'une quelconque des revendications 1 et 2, caractérisée en ce que des boues de marais, des boues pour bains, de la terre glaise (terre curative) ou analogues sont utilisées comme masse de la nature de la terre, en particulier sous une forme en substance sèche.

4. Poche chauffante selon l'une quelconque des revendications 1 à 3, caractérisée en ce que de la paraffine sert de masse à fondre.

5. Poche chauffante selon l'une quelconque des revendications 1 à 4, caractérisée en ce que la couche (4) située entre les deux couches extérieures (2, 5) se compose de 30 à 70 % en poids de la masse de la nature de la terre et de 70 à 30 % en poids de la masse à fondre, et a de préférence un rapport de mélange de 1/1.

6. Poche chauffante selon l'une quelconque des revendications 1 à 5, caractérisée en ce que la couche (4) formée de la masse de la nature de la terre et de la masse à fondre est présente, entre les deux couches extérieures (2, 5), selon un poids de 3 à 30 kg par m², de préférence de 8 à 15 kg par m².

7. Poche chauffante selon l'une quelconque des revendications 1 à 6, caractérisée en ce que des matières actives telles que des herbes médicinales, des huiles, du soufre, des extraits de plantes ou analogues sont mélangées avec la couche (4) constituée de la masse de la nature de la terre et de la masse à fondre.

8. Poche chauffante selon l'une quelconque des revendications 1 à 7, caractérisée en ce qu'au moins la couche extérieure (5) se compose d'une nappe de fibres activement aiguilletables.

9. Poche chauffante selon l'une quelconque des revendications 1 à 8, caractérisée en ce que la nappe de fibres (5) a un poids de 20 à 350 g par m², de préférence de 100 à 180 g par m².

10. Poche chauffante selon l'une quelconque des revendications 8 et 9, caractérisée en ce que l'autre couche extérieure (2) se compose également d'une nappe de fibres et a de préférence un poids de 100 à 180 g par m².

11. Poche chauffante selon l'une quelconque des revendications 1 à 9, caractérisée en ce que la deuxième couche extérieure (2') se compose d'une feuille et a de préférence un poids de 50 à 200 g par m².

12. Poche chauffante selon la revendication 11, caractérisée en ce que la feuille (2') est une feuille d'aluminium.

13. Poche chauffante selon l'une quelconque des revendications 11 et 12, caractérisée en ce que la feuille (2') est une feuille à godets.

14. Poche chauffante selon l'une quelconque des revendications 1 à 13, caractérisée en ce que la fraction de la masse (17) de la nature de la terre est plus grande au voisinage de la couche extérieure (5), au moins perméable aux liquides, qu'au voisinage de l'autre couche extérieure (2).

15. Poche chauffante selon l'une quelconque des revendications 1 à 14, caractérisée en ce que les fibres de retenue (19) sont utilisées selon une densité superficielle telle qu'elles empêchent un glissement de la masse à fondre et de la masse de la nature de la terre.

16. Poche chauffante selon l'une quelconque des revendications 1 à 15, caractérisée en ce que les fibres de retenue (19) rapprochent l'une de l'autre les deux couches extérieures (2, 5) de telle sorte que celles-ci exercent une pression sur la couche (4) constituée de la masse de la nature de la terre et de la masse à fondre.

17. Poche chauffante selon l'une quelconque des revendications 1 à 16, caractérisée en ce que le système à trois couches (2, 4, 5) présente, sur ses bords périphériques (23), une épaisseur plus petite que celle éloignée desdits bords (23).

18. Poche chauffante selon l'une quelconque des revendications 1 à 17, caractérisée en ce que la masse de la nature de la terre se présente sous une forme granulée.

19. Poche chauffante selon l'une quelconque des revendications 1 à 18, caractérisée en ce que la masse de la nature de la terre se présente sous une forme en substance sèche.

20. Poche chauffante selon l'une quelconque des revendications 1 à 19, caractérisée en ce que la masse à fondre se présente sous une forme granulée.

21. Poche chauffante selon l'une quelconque des revendications 1 à 19, caractérisée en ce que la masse à fondre se présente comme une couche continue, pénétrée par les fibres de retenue.

22. Poche chauffante selon l'une quelconque des revendications 1 à 21, caractérisée en ce que la masse de la nature de la terre est enrobée dans la masse à fondre.

23. Poche chauffante selon l'une quelconque des revendications 1 à 22, caractérisée en ce que la poche formée des couches extérieures (2, 5) et de la couche (4) constituée de la masse à fondre et de la masse de la nature de la terre est disposée entre deux feuilles (15, 16) étanches aux gaz et aux liquides et en ce que lesdites feuilles (15, 16) sont reliées l'une à l'autre d'une manière étanche par leurs bords (24).

24. Poche chauffante selon l'une quelconque des revendications 1 à 23, caractérisée en ce qu'un vide est présent entre ces deux feuilles (15, 16).

25. Procédé de fabrication d'une poche chauffante dans laquelle une masse de la nature de la terre est disposée entre deux couches extérieures planes (2, 5) enrobant la masse, et lesdites deux couches (2, 5) sont reliées l'une à l'autre au moins par leurs bords, caractérisé en ce qu'une couche (4) constituée d'une masse de la nature de la terre et d'une masse à fondre composée de cires d'hydrocarbures synthétiques et/ou naturelles est insérée entre les deux couches extérieures (2, 5) et en ce que ces deux couches (2, 5) entourant la couche intérieure (4) sont reliées l'une à l'autre à l'aide de fibres de retenue (19) réparties sur toute la surface et passant à travers la couche intérieure (4).

26. Procédé selon la revendication 25, caractérisé en ce qu'une couche activement aiguilletable est utilisée au moins pour l'une deux couches extérieures et en ce que ces deux couches extérieures sont aiguilletées l'une avec l'autre par l'intermédiaire de la couche intérieure présente sous une forme plane.

27. Procédé selon l'une quelconque des revendications 25 et 26, caractérisé en ce que les trois couches reliées les unes aux autres par les fibres de retenue sont partagées en grandeurs superficielles conformes aux utilisations et en ce qu'une poche ainsi réalisée est emballée entre deux feuilles imperméables aux gaz et à l'eau.

28. Procédé selon la revendication 27, caractérisé en ce que le partage en grandeurs superficielles conformes aux utilisations est effectué au moyen d'outils chauds et en ce que les trois couches sont pressées mécaniquement les unes contre les autres, simultanément et directement à côté de la ligne de coupe.

29. Procédé selon l'une quelconque des revendications 27 et 28, caractérisé en ce que la poche est emballée sous vide entre les deux feuilles.

# Fig. 1

**Fig. 2**

**Fig. 3**

**Fig. 4**